# EUROPEAN PATENT SPECIFICATION

(11) **EP 4 023 077 B1**
(45) Date of publication and mention of the grant of the patent: **02.08.2023**
(21) Application number: 21753804.0
(22) Date of filing: 07.02.2021
(51) Int. Cl.: A23L 33/125, A23L 33/135, A23L 33/15, A23P 20/12

(54) **ENTERAL SUSTAINED-RELEASE SUGAR ALCOHOL ADDITIVE, PREPARATION METHOD THEREFOR AND APPLICATION THEREOF**
ENTERALER ZUCKERALKOHOLZUSATZ MIT VERZÖGERTER FREISETZUNG, HERSTELLUNGSVERFAHREN DAFÜR UND ANWENDUNG DAVON
ADDITIF ENTÉRAL À BASE D'ALCOOL DE SUCRE À LIBÉRATION PROLONGÉE, SON PROCÉDÉ DE PRÉPARATION ET SON APPLICATION

(30) Priority: 11.02.2020 CN 202010087312
(43) Date of publication of application: 06.07.2022
(73) Proprietor: Zhejiang Huakang Pharmaceutical Co., Ltd., Quzhou, Zhejiang 324302 (CN)
(72) Inventor: XIANG, Shasha, Quzhou, Zhejiang 324302 (CN); ZHU, Xuan, Quzhou, Zhejiang 324302 (CN); SHI, Lihua, Quzhou, Zhejiang 324302 (CN); YE, Kun, Quzhou, Zhejiang 324302 (CN); LI, Mian, Santa Clara, California 95054 (US)
(74) Representative: Sun, Yiming
(86) International application number: PCT/CN2021/075696
(87) International publication number: WO 2021/160054

(56) References cited:
- WO-A1-2007/110645
- WO-A1-2014/082132
- WO-A1-2019/209261
- CN-A- 101 541 194
- CN-A- 104 936 464
- CN-A- 106 820 069
- CN-A- 107 259 577
- CN-A- 107 530 381
- CN-A- 108 576 895
- CN-A- 111 280 449
- US-A1- 2017 165 201
- Sun Jianhui: "Therapeutic effects of vitamin B12", Knowledge of Cardiovascular Disease Prevention and Treatment, 1 April 2008 (2008-04-01), pages 54-57, XP055835276,

## Description

### TECHNICAL FIELD

The present invention relates to the field of preparation technologies of enteral sustained-release products, in particular to an enteral sustained-release sugar alcohol additive and a preparation method and an application thereof.

### BACKGROUND

After being absorbed, propionate is mainly metabolized in liver to participate in gluconeogenesis and inhibit synthesis of cholesterol. However, various researches show that propionates ingested exogenously and generated in body have different effects. The exogenous propionate has significant impact on many metabolic processes of the individuals. Propionate is a short-chain fatty acid. Under normal circumstances, microorganisms in a human body are capable of generating propionate by fermenting un-completely digested carbohydrate in colon and the propionate is beneficial to the human body. But, the exogenous propionate may lead to hyperglycemia and a high level of insulin in a short period, and may even lead to more severe symptoms such as obesity and insulin resistance or the like after a long period of ingestion.

Fructo-oligosaccharide and polysaccharide have the function of generating propionate in intestinal tract. But, neither of fructo-oligosaccharide and polysaccharide can synthesize propionate with a high concentration at a fixed point. The enteral utilization rate of polysaccharide is low and may easily lead to problems of flatulence and the like. Fructo-oligosaccharide may also lead to the problems of excess generation of gas and unstable generation of acid and the like, and even to metabolic disorder of liver and the like due to excess generation of fructose. CN 107530381 A discloses a composition for reducing intestinal gas production. CN 107259577 A discloses a probiotic gel grain. US 2017/0165201 A1 discloses pH-responsive mucoadhesive polymeric encapsulated microorganisms. WO 2014/082132 A1 discloses probiotic-containing microparticles. WO 2019/209261 discloses protected compositions comprising two coating layers.

### SUMMARY

### Technical problem

In order to solve the above technical problems, the present invention provides an enteral sustained-release sugar alcohol additive and a preparation method and an application thereof so as to promote human body health and eliminate metabolic side effect possibly resulting from propionate, thereby achieving slow release of the sugar alcohol additive in intestinal tract and its fixed-point release in colon.

### Solution to problems

The present invention is achieved by providing an enteral sustained-release sugar alcohol additive. The enteral sustained-release sugar alcohol additive includes an inner layer structure and an outer layer structure, wherein the inner layer structure contains components of xylitol and agar, and the outer layer structure contains components of carrageenan or gellan gum or xanthan gum or guar gum, vitamin B12, L-arabinose, and fermented bifidobacterium or fermented propionibacterium or fermented lactobacillus.

The present invention is achieved by further providing a method of preparing the enteral sustained-release sugar alcohol additive as described above. The method includes the following steps:

at step 1, mixing and sterilizing xylitol and agar, placing the mixture into a ball mould and standing the mixture for cooling so as to prepare xylitol agar pellets;

at step 2, preparing a carrageenan solution or gellan gum solution or xanthan gum solution or guar gum solution, adding vitamin B12 and L-arabinose to the carrageenan solution or gellan gum solution or xanthan gum solution or guar gum solution and mixing to uniformity so as to obtain a mixed solution, and then wrapping the xylitol agar pellets in step 1 with the mixed solution to obtain xylitol agar two-layer colloidal pellets wrapped with the mixed solution;

at step 3, placing the two-layer colloidal pellets prepared in step 2 into a fermented bifidobacterium medium, or a fermented propionibacterium medium or a fermented lactobacillus medium for culturing, and then taking out the pellets for freeze or hot air drying to obtain a finished sugar alcohol additive.

The present invention is achieved by further providing an application of the enteral sustained-release sugar alcohol additive as described above. The sugar alcohol additive is applied to foodstuff.

The present invention is achieved by further providing an application of an enteral sustained-release sugar alcohol additive prepared using the above method of preparing an enteral sustained-release sugar alcohol additive. The sugar alcohol additive is applied to foodstuff.

### Beneficial effects of the invention

Compared with the prior art, the enteral sustained-release sugar alcohol additive and the preparation method and the application thereof in the present invention have the following advantages.

1. With xylitol, L-arabinose and vitamin B12, directional regulation to intestinal microorganisms is achieved and enteral high-concentration synthesis of propionate can be realized.

2. With double layer embedding technology, enteral sustained release and layer-by-layer release of sugar alcohol can be achieved.

3. With the pellet technology of the method, stability of bifidobacterium or propionibacterium or lactobacillus products is achieved.

### BRIEF DESCRIPTION OF THE DRAWINGS

FIG. 1 is a schematic diagram illustrating a change of a content of xylitol in an enteral sustained-release sugar alcohol additive in a simulation digestion process of stomach, small intestine and colon according to the present invention.
FIG. 2 is a schematic diagram illustrating a change of a content of L-arabinose in an enteral sustained-release sugar alcohol additive in a simulation digestion process of stomach, small intestine and colon according to the present invention.
FIG. 3 is a comparative schematic diagram of relative abundances of bifidobacterium of a two-layer colloidal pellet product and a finished enteral sustained-release sugar alcohol additive in a simulation colon experiment according to the present invention.

### DETAILED DESCRIPTION OF THE EMBODIMENTS

In order to make the technical problems, technical solutions and beneficial effects of the present invention clearer, the present invention will be further described below in combination with the accompanying drawings and the specific embodiments. It should be understood that the specific embodiments described herein are merely used to explain the present invention rather than limit the present invention.

An enteral sustained-release sugar alcohol additive according to the present invention includes an inner layer structure and an outer layer structure, where the inner layer structure contains components of xylitol and agar, and the outer layer structure contains components of carrageenan or gellan gum or xanthan gum or guar gum, vitamin B12, L-arabinose, and fermented bifidobacterium or fermented propionibacterium or fermented lactobacillus.

Specifically, in the sugar alcohol additive, a component ratio of xylitol to L-arabinose to vitamin B12 is 1-33:1:0.005.

In the enteral sustained-release sugar alcohol additive of the present invention, by compounding xylitol, L-arabinose and vitamin B12, L-arabinose slows down metabolism of xylulose to arabitol and controls metabolic flux, vitamin B12 increases propionate metabolism pathways. In this way, increased gene expressions of enterobacterium and lachnospiraceae relating to xylitol are induced firstly, thereby increasing metabolism of xylitol to PPP pathway, weakening the pathway in which xylulose is metabolized to arabitol and increasing propionate synthesis amount.

In the present invention, the enteral sustained-release sugar alcohol additive adopts two layers of structures. The two-layer colloid prepared using hydrocolloid such as carrageenan and L-arabinose and vitamin B12 can realize sustained release of xylitol and L-arabinose in intestinal tract and their fixed-point release in colon.

The present invention further provides a method of preparing the enteral sustained-release sugar alcohol additive as described above. The method includes the following steps.

At step 1, xylitol and agar are mixed and sterilized and then placed into a ball mould for standing cooling, so as to prepare xylitol agar pellets.

At step 2, a carrageenan solution or gellan gum solution or xanthan gum solution or guar gum solution is prepared, vitamin B12 and L-arabinose are added to the carrageenan solution or gellan gum solution or xanthan gum solution or guar gum solution and then mixed to uniformity so as to obtain a mixed solution, and then the xylitol agar pellets in step 1 are wrapped with the mixed solution to obtain xylitol agar two-layer colloidal pellets wrapped with the mixed solution.

At step 3, the two-layer colloidal pellets prepared in step 2 are placed into a fermented bifidobacterium medium, or a fermented propionibacterium medium or a fermented lactobacillus medium for culturing, and then taken out for freeze or hot air drying to obtain a finished sugar alcohol additive.

### Embodiments of the invention

The method of preparing an enteral sustained-release sugar alcohol additive according to the present invention will be further described below in combination with specific embodiments.

### Embodiment 1

According to a first embodiment of the present invention, the method of preparing an enteral sustained-release sugar alcohol additive may include the following steps.

At step 1, a given amount of xylitol was weighed and mixed with 2% agar, and then sterilized for 15min at a temperature of 121°C; when the mixture was cooled down to 55°C, the mixture was transferred to a ball mould by sucking using a disposable aseptic rubber head dropper and then stood and cooled down so as to prepare xylitol agar pellets.

At step 2, 2g of carrageenan was weighed and dissolved in 100ml of distilled water and then sterilized for 15min at a temperature of 121 °C and then cooled down to 50°C-60°C with the temperature no greater than 60°C to help vitamin B12 to stabilize and avoid light, and aseptic L-arabinose and vitamin B12 were added at a formulation ratio of 1:0.005 to the carrageenan solution and then mixed to uniformity; then the xylitol agar pellets were wrapped using the above mixed solution in an aseptic super-clean table to prepare two-layer colloidal pellets with a formulation ratio of 1-33:1:0.005.

At step 3, the two-layer colloidal pellets prepared at step 2 were added to a bifidobacterium fermentation (10⁹CFU/mL) broth at a ratio of 1:3 of total volume and stood for culture of 24 hours; then the pellets with bacteria were taken out and then subjected to freeze drying for 1h-24h or hot air drying for 30min so as to obtain a finished sugar alcohol additive.

A new embodiment may be obtained by replacing bifidobacterium with propionibacterium or lactobacillus in the above embodiment.

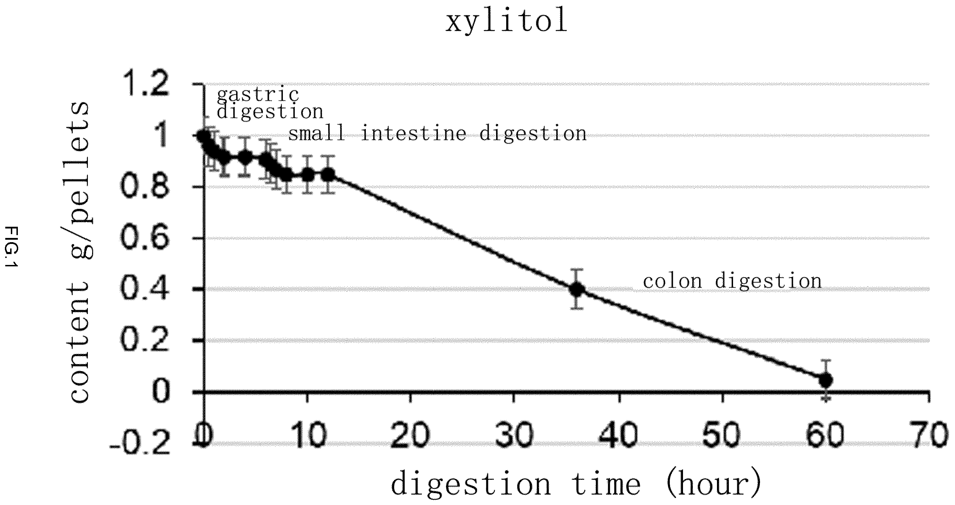

The enteral sustained-release sugar alcohol additive prepared in the embodiment is selected to perform simulation digestion experiment of stomach, small intestine and colon for verification in the following method.
1) Preparation of simulation gastric fluid: 0.62g of NaCl, 0.22g of KCl, 0.05g of CaCl₂, and 0.12g of NaHCO₃ were dissolved in 200ml of distilled water, and adjusted to pH 2.0 using 0.1M HCl solution to prepare a gastric fluid medium. Then, 1.0mL of CH₃COONa(1.0mol/L, pH5), 23.6g of pepsase and 25.0g of gastric lipase were added to 100ml of the gastric medium and then the pH value of the gastric medium was adjusted to 2.0 using 0.1M HCl solution.
2) Simulation of gastric fluid digestion: based on a dose of 1%-5% of xylitol, the finished sugar alcohol additive pellets of the embodiment 1 were mixed with the simulation gastric fluid at a volume ratio of 1:1 and then subjected to water bath for 0.5h, 1h, 2h, 4h and 6h at a temperature of 37°C to respectively obtain digested samples.
3) Preparation of simulation small intestine digestive fluid: 0.54g of NaCl, 0.065g of KCl and 0.033g of CaCl₂ were dissolved in 100ml of distilled water and adjusted to pH 7.0 using 0.1M NaOH solution to prepare a small intestine medium. Then, 6.5mg of trypsin, 200.0g of bile salt (4%, w/w) and 50.0g of trypsin solution (7%, w/w) were added to the small intestine medium respectively, and then stirred continuously at room temperature using 0.1M NaOH solution to adjust the solution pH to 7.5 so as to simulate a small intestine digestive fluid.
4) Simulation of small intestine digestion: the samples after simulation of gastric fluid digestion were mixed with the small intestine fluid respectively at a volume ratio of 3:7 and then subjected to water bath for 0.5h, 1h, 2h, 4h, and 6h at a temperature of 37°C so as to obtain digested samples respectively.
5) Based on a dose of 1%-5% of xylitol, the two-layer colloidal pellet samples (without bifidobacterium embedded) and the finished sugar alcohol additive pellet samples (with bifidobacterium embedded) in the embodiment 1 were supplemented to CDMN (Chang Dao Mo Ni) colon in vitro simulation system to collect pellet samples simulating colon digestion of 24h and 48h.
6) With HPLC, contents of xylitol and arabinose and an abundance value of bifidobacterium retained in the pellets after simulation of gastric digestion, small intestine digestion and colon digestion were measured respectively to obtain comparison curves of FIGS. 1-3.

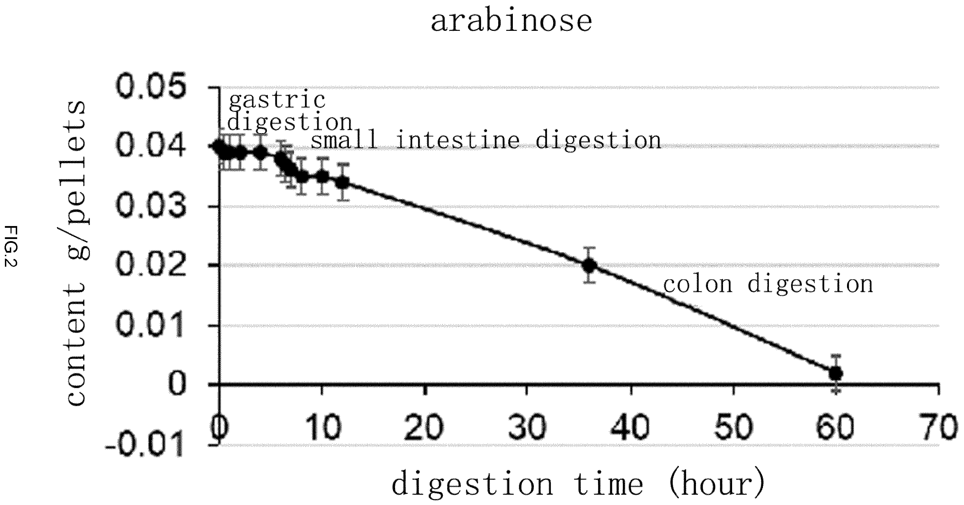

As shown in FIGS. 1 and 2, with xylitol: arabinose=25:1 as an example, in each pellet, the content of xylitol is 1g, and the content of arabinose is 0.04g. The first 6 hours relate to gastric digestion, the 6-12 hours relate to digestion of small intestine, and the 12-60 hours relate to contents of xylitol and arabinose retained in the pellets after microbial digestion of large intestine. Compared with the existing experimental data (the absorption rate of the small intestine for xylitol is 60% and the absorption rate for arabinose is 70%), the absorption rate of xylitol is only 15% and the absorption rate of arabinose is only 25% in the pellets of the present invention, and their cumulative release rates in the colon are about 95% respectively. It indicates that the pellets of the present invention have the effect of slow and fixed-point release for xylitol and arabinose.

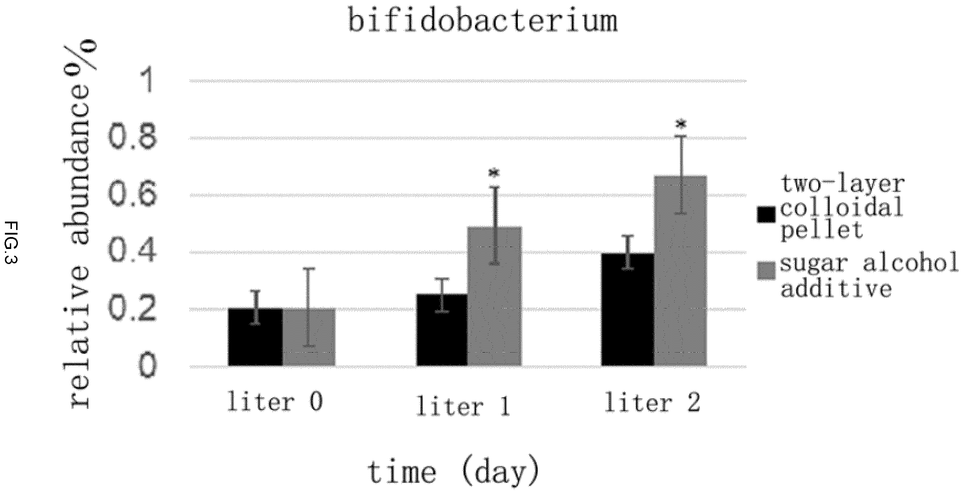

As shown in FIG. 3, compared with non-embedding (two-layer colloidal pellet product), the embedding of bifidobacterium (finished sugar alcohol additive) in the pellets of the present invention can obviously promote the relative abundance of bifidobacterium in the pellets, thereby promoting the relative abundance of bifidobacterium in the colon.

As a result, the enteral sustained-release sugar alcohol additive of the present invention adopts layer-by-layer embedding technology to realize slow and layer-by-layer release of xylitol in intestinal tract and promote the relative abundance of bifidobacterium in the colon.

The present invention further provides an application of the enteral sustained-release sugar alcohol additive as described above. The sugar alcohol additive is applied to foodstuff.

For example, the enteral sustained-release sugar alcohol additive of the present invention is applied to beverage and cakes to promote generation of propionate in the colon of the human body and adjust intestinal health.

## Claims

1. An enteral sustained-release sugar alcohol additive, comprising an inner layer structure and an outer layer structure, wherein the inner layer structure contains components of xylitol and agar, and the outer layer structure contains components of carrageenan or gellan gum or xanthan gum or guar gum, vitamin B12, L-arabinose, and fermented bifidobacterium or fermented propionibacterium or fermented lactobacillus.

2. The enteral sustained-release sugar alcohol additive of claim 1, wherein in the sugar alcohol additive, a component ratio of xylitol to L-arabinose to vitamin B12 is 1-33: 1:0.005.

3. A method of preparing the enteral sustained-release sugar alcohol additive according to claim 1 or 2, comprising the following steps:
at step 1, mixing and sterilizing xylitol and agar, placing the mixture into a ball mould and standing the mixture for cooling so as to prepare xylitol agar pellets;
at step 2, preparing a carrageenan solution or gellan gum solution or xanthan gum solution or guar gum solution, adding vitamin B12 and L-arabinose to the carrageenan solution or gellan gum solution or xanthan gum solution or guar gum solution and mixing to uniformity so as to obtain a mixed solution, and then wrapping the xylitol agar pellets in step 1 with the mixed solution to obtain xylitol agar two-layer colloidal pellets wrapped with the mixed solution;
at step 3, placing the two-layer colloidal pellets prepared in step 2 into a fermented bifidobacterium medium, or a fermented propionibacterium medium or a fermented lactobacillus medium for culturing, and then taking out the pellets for freeze or hot air drying to obtain a finished sugar alcohol additive.

4. The method of claim 3, wherein in step 1, a concentration of agar is 2%, and a sterilization condition is sterilization of 15min at a temperature of 121°C.

5. The method of claim 3, wherein in step 2, the method of preparing the carrageenan solution or gellan gum solution or xanthan gum solution or guar gum solution includes: weighing 2g of carrageenan or gellan gum or xanthan gum or guar gum and dissolving in 100ml of distilled water, sterilizing for 15min at a temperature of 121 °C and cooling down to a temperature of 50°C-60°C.

6. The method of claim 3, wherein in step 3, a concentration of bifidobacterium or propionibacterium or lactobacillus in the fermented bifidobacterium medium, or the fermented propionibacterium medium or the fermented lactobacillus medium is 10⁹CFU/mL, the two-layer colloidal pellets are placed in the fermented bifidobacterium medium, or the fermented propionibacterium medium or the fermented lactobacillus medium for culturing for 24h, wherein the condition of freeze or hot air drying is freeze drying for 1h-24h or hot air drying for 30min.

7. An application of the enteral sustained-release sugar alcohol additive according to 1 or 2, wherein the sugar alcohol additive is applied to foodstuff.

## Patentansprüche

1. Zuckeralkohol-Additiv mit verzögerter enteraler Wirkstofffreigabe, umfassend eine Innnenschichtstruktur und eine Außenschichtstruktur, wobei die Innnenschichtstruktur Komponenten von Xylitol und Agar enthält, und die Außenschichtstruktur Komponenten von Carrageen oder Gellangummi oder Xanthangummi oder Guargummi, Vitamin B12, L-Arabinose, und fermentiertem Bifidobacterium oder fermentiertem Propionibacterium oder fermentiertem Lactobacillus enthält.

2. Zuckeralkohol-Additiv mit verzögerter enteraler Wirkstofffreigabe nach Anspruch 1, wobei in dem Zuckeralkohol-Additiv das Komponentenverhältnis von Xylitol zu L-Arabinose zu Vitamin B12 1-33:1:0,005 ist.

3. Verfahren zum Herstellen des Zuckeralkohol-Additivs mit verzögerter enteraler Wirkstofffreigabe nach Anspruch 1 oder 2, umfassend die folgenden Schritte:
Schritt 1: Mischen und Sterilisieren von Xylitol und Agar, Einbringen der Mischung in eine Kugel-Gießform und Stehenlassen der Mischung zum Abkühlen, um so Xylitol-Agar-Pellets anzufertigen;
Schritt 2: Anfertigen einer Carrageen-Lösung oder Gellangummi-Lösung oder Xanthangummi-Lösung oder Guargummi-Lösung, Hinzufügen von Vitamin B12 und L-Arabinose zu der Carrageen-Lösung oder Gellangummi-Lösung oder Xanthangummi-Lösung oder Guargummi-Lösung und Mischen bis zu Gleichförmigkeit, um so eine gemischte Lösung zu erhalten, und dann Einhüllen der Xylitol-Agar-Pellets von Schritt 1 in der gemischten Lösung, um kolloide Xylitol-Agar-Pellets mit zwei Schichten zu erhalten, die in der gemischten Lösung eingehüllt sind;
Schritt 3: Einbringen der in Schritt 2 angefertigten kolloiden Zwei-Schicht-Pellets in ein fermentiertes Bifidobacterium-Medium oder ein fermentiertes Propionibacterium-Medium oder ein fermentiertes Lactobacillus-Medium zum Kultivieren, und dann Entnehmen der Pellets zum Gefrier- oder Heißlufttrocknen, um ein fertiggestelltes Zuckeralkohol-Additiv zu erhalten.

4. Verfahren nach Anspruch 3, wobei in Schritt 1 die Konzentration von Agar 2 % beträgt, und die Sterilisierungsbedingung eine Sterilisierung von 15 min bei einer Temperatur von 121 °C ist.

5. Verfahren nach Anspruch 3, wobei in Schritt 2 das Verfahren zum Herstellen der Carrageen-Lösung oder Gellangummi-Lösung oder Xanthangummi-Lösung oder Guargummi-Lösung umfasst: Abwiegen von 2 g Carrageen oder Gellangummi oder Xanthangummi oder Guargummi und Auflösen in 100 ml destilliertem Wasser, Sterilisieren über 15 min bei einer Temperatur von 121 °C und Abkühlen auf eine Temperatur von 50 °C - 60 °C.

6. Verfahren nach Anspruch 3, wobei in Schritt 3 die Konzentration von Bifidobacterium oder Propionibacterium oder Lactobacillus in dem fermentierten Bifidobacterium-Medium oder dem fermentierten Propionibacterium-Medium oder dem fermentierten Lactobacillus-Medium 10⁹KBE/ml ist, die kolloiden Zwei-Schicht-Pellets in das fermentierte Bifidobacterium-Medium oder das fermentierte Propionibacterium-Medium oder das fermentierte Lactobacillus-Medium zum Kultivieren über 24 h eingebracht werden, wobei die Bedingung des Gefrier- oder Heißlufttrocknens ein Gefriertrocknen über 1 h - 24 h oder ein Heißlufttrocknen über 30 min ist.

7. Anwendung des Zuckeralkohol-Additivs mit verzögerter enteraler Wirkstofffreigabe nach Anspruch 1 oder 2, wobei das Zuckeralkohol-Additiv auf Nahrungsmittel angewendet wird.

## Revendications

1. Additif alcoolisé sucré à libération prolongée entérique, comprenant une structure de couche interne et une structure de couche externe, dans lequel la structure de couche interne contient des composants de xylitol et d'agar et la structure de couche externe contient des composants de carraghénane ou de gomme de gellane ou de gomme de xanthane ou de gomme de guar, de la vitamine B12, du L-arabinose et du bifidobacterium fermenté ou du propionibacterium fermenté ou du lactobacillus fermenté.

2. Additif alcoolisé sucré à libération prolongée entérique selon la revendication 1, dans lequel dans l'additif alcoolisé sucré, un rapport de composants de xylitol à L-arabinose à vitamine B12 est de 1 à 33:1:0,005.

3. Procédé de préparation de l'additif alcoolisé sucré à libération prolongée entérique selon la revendication 1 ou 2, comprenant les étapes suivantes consistant à :
à l'étape 1, mélanger et stériliser le xylitol et l'agar, placer le mélange dans un moule à boule et laisser reposer le mélange pour le refroidir de manière à préparer des pastilles de xylitol agar ;
à l'étape 2, préparer une solution de carraghénane ou un solution de gomme de gellane ou une solution de gomme de xanthane ou une solution de gomme de guar, ajouter la vitamine B12 et le L-arabinose à la solution de carraghénane ou la solution de gomme de gellane ou la solution de gomme de xanthane ou la solution de gomme de guar et mélanger à uniformité pour obtenir une solution mixte, puis enrouler les pastilles de xylitol agar dans l'étape 1 avec la solution mixte pour obtenir des pastille colloïdales à deux couches de xylitol agar enroulées avec la solution mixte ;
à l'étape 3, placer les pastilles colloïdales à deux couches préparées dans l'étape 2 dans un milieu de bifidobacterium fermenté ou un milieu de propionibacterium fermenté ou un milieu de lactobacillus fermenté pour la culture, puis prélever les pastilles pour la congélation ou le séchage à l'air chaud pour obtenir un additif alcoolisé sucré fini.

4. Procédé selon la revendication 3, dans lequel dans l'étape 1, une concentration d'agar est de 2 % et une condition de stérilisation est une stérilisation de 15 minutes à une température de 121 °C.

5. Procédé selon la revendication 3, dans lequel dans l'étape 2, le procédé de préparation de la solution de carraghénane ou de la solution de gomme de gellane ou de la solution de gomme de xanthane ou de la solution de gomme de guar comprend : la pesée de 2 grammes de carraghénane ou de gomme de gellane ou de gomme de xanthane ou de gomme de guar et la dissolution dans 100 ml d'eau distillée, la stérilisation durant 15 minutes à une température de 121 °C et le refroidissement à une température de 50 à 60 °C.

6. Procédé selon la revendication 3, dans lequel dans l'étape 3, une concentration de bifidobacterium ou de propionibacterium ou de lactobcillus dans le milieu de bifidobacterium fermeté ou le milieu de propionibactrium fermenté ou le milieu de lactobacillus fermenté est de 10⁹ UFC/mL les pastilles colloïdales à deux couches sont placées dans le milieu de bifidobacterium fermenté ou le milieu de propionibacterium fermenté ou le milieu de lactobacillus fermenté pour être cultivées durant 24 h, la condition de congélation ou de séchage à l'air chaud étant un séchage par congélation durant 1 h à 24 h ou un séchage à l'air chaud durant 30 minutes.

7. Application de l'additif alcoolisé sucré à libération prolongée entérique selon la revendication 1 ou 2, dan laquelle l'additif alcoolisé sucré est appliqué à un aliment.
